# EUROPÄISCHE PATENTSCHRIFT

(11) **EP 4 353 331 B1**
(45) Veröffentlichungstag und Bekanntmachung des Hinweises auf die Patenterteilung: **04.03.2026**
(21) Anmeldenummer: 23198333.9
(22) Anmeldetag: 19.09.2023
(51) Int. Cl.: A62C 99/00, A62C 3/04, B23Q 11/00

(54) **VORRICHTUNG UND VERFAHREN ZUR ÜBERWACHUNG EINER INERTISIERUNG**
DEVICE AND METHOD FOR MONITORING INERTING
DISPOSITIF ET PROCÉDÉ DE SURVEILLANCE D'UN INERTAGE

(30) Priorität: 13.10.2022 DE 102022126773
(43) Veröffentlichungstag der Anmeldung: 17.04.2024
(73) Patentinhaber: Pilz GmbH & Co. KG, 73760 Ostfildern (DE)
(72) Erfinder: Ossapofsky, Reinhold-Johannes, 73760 Ostfildern (DE)
(74) Vertreter: Witte, Weller & Partner Patentanwälte mbB

(56) Entgegenhaltungen:
- DE-A1- 102017 123 999
- US-A1- 2008 057 686
- US-A1- 2018 239 374

## Beschreibung

Die vorliegende Erfindung betrifft eine Vorrichtung und ein Verfahren zur Überwachung einer Inertisierung.

Inertisierung bezeichnet allgemein einen Vorgang, bei dem durch die Zugabe eines Inertgases, d. h. durch Hinzugabe eines reaktionsträgen Gases, zu einem Volumen, die Bildung eines explosionsfähigen Gemisches innerhalb des Volumens verhindert wird. Die Inertisierung von Räumen zum Brand- und Explosionsschutz ist grundsätzlich bekannt und wird unter anderem bei Chemikalienlagern, Produktionsanlagen und im Flugzeugbau eingesetzt.

DE 10 2008 013 150 A1 zeigt beispielsweise ein System zur Inertisierung eines Gasvolumens bei einem Flugzeug, bei dem zwei Abgase zu einem Inertgas vermischt und anschließend einem zu inertisierenden Gasvolumen hinzugefügt werden. Das Inertgas verdrängt oder verdünnt reaktionsfreudige Gase in dem Gasvolumen, sodass von diesen keine Gefahr mehr ausgeht. Ob eine ausreichende Inertisierung stattgefunden hat, überprüft das System über eine Messsonde, die in dem zu inertisierenden Gasvolumen angeordnet ist und die chemische und stoffliche Zusammensetzung des Gasvolumens nach Einleiten des Inertgases bestimmt. Entsprechen die bestimmten Gase in dem inertisierten Gasvolumen einem definierten Verhältnis, wird von einer ausreichenden Inertisierung ausgegangen.

Zur Fertigung von Halbleiterbauelementen werden in der Halbleiterindustrie spezielle Fertigungsanlagen eingesetzt, die in der Fachsprache auch als Tools bezeichnet werden. Die Fertigungsanlagen haben eine abgeschlossene Kammer, innerhalb derer der Aufbau des Halbleiterelementes stattfindet. In die Kammer werden für verschiedene Arbeitsschritte Prozessgase eingeleitet, die entweder unmittelbar dem Aufbau des Halbleiterelements dienen oder eine für die Fertigung benötigte Fertigungsumgebung bereitstellen. Die Prozessgase werden nach Abschluss eines Fertigungsschritts oder nach Fertigstellung des Halbleiterelements aus der Kammer entfernt und in einen Abgastrakt überführt.

Die Prozessgase können giftige oder stark reaktionsfreudige Gase beinhalten, die im Abgastrakt ausreichend neutralisiert werden müssen. Beispielsweise kann Wasserstoff als Prozessgas verwendet werden, das sich mit dem Sauerstoff der Luft vermischt und bei Entzündung eine Knallgasreaktion auslöst. Je nachdem, welche Rezepte und integrierte Sicherheitsfunktionen ein Tool mitbringt, kann der eingeleitete Wasserstoff bis zu 100% am Ausgang des Tools wieder abgeleitet werden. Da nicht alle Halbleiterfabriken (Fabs) einen explosionsgeschützten Abgastrakt (Exhaust) aufweisen oder die Tools selbst für eine Beseitigung von Restwasserstoff konzipiert sind, müssen Maßnahmen zum Schutz von Menschen und Maschinen ergriffen werden, um ein von reaktionsfreudigen Gasen ausgehendes Explosionsrisiko zu beseitigen.

Eine Möglichkeit hierfür ist, eine Inertisierung innerhalb des Abgastrakts durchzuführen, bei der in Form einer Spülung (sog. Purging) der abgeleitete Wasserstoff mit ausreichend Inertgas (bspw. Stickstoff) verdünnt wird bzw. der möglicherweise vorhandene Sauerstoff verdrängt wird, sodass eine Wasserstoff-Sauerstoff-Konzentration nicht das Knallgas Volumenverhältnis von 2:1 (H₂:O₂) überschreitet und kein Risiko für eine Knallgasreaktion mehr besteht. Bei nicht-intelligenten Systemen wird hierzu die größtmögliche Menge an eingeleiteten Wasserstoff bestimmt und basierend hierauf eine entsprechende Menge Inertgas dem Abgastrakt hinzugegeben, sodass auch in dem Fall, dass bis zu 100% des eingeleiteten Wasserstoffs in den Abgastrakt abgeführt werden, eine ausreichende Inertisierung stattfinden kann. Es versteht sich, dass bei diesem statischen Verfahren eine unnötig große Menge an Inertgas verbraucht wird. Intelligente Systeme wiederum passen die benötigte Menge an Inertgas an die tatsächliche Menge an eingeleiteten bzw. abgeführten Wasserstoff dynamisch an. Somit wird für die Inertisierung nur die tatsächlich notwendige Menge an Inertgas in den Abgastrakt eingeleitet, wodurch sich die Inertisierung effizienter realisieren lässt. Für einen sicheren Betrieb einer dynamischen Inertisierung ist es jedoch notwendig, dass die Inertisierung kontinuierlich überwacht und bei Fehlverhalten eine entsprechende Reaktion ausgelöst wird, die die Anlage oder den Abgastrakt in einen sicheren Zustand überführt.

Eine solche Überwachung setzt jedoch voraus, dass die verschiedenen Gase oder zumindest die Gasanteile, die durch die Inertisierung verdünnt oder verdrängt werden sollen, hinreichend bestimmt werden können. In der Praxis hat sich jedoch gezeigt, dass eine Sonde in dem zu inertisierenden Volumen, wie sie beispielsweise in der eingangs genannten DE 10 2008 013 150 A1 gezeigt ist, nicht ohne Weiteres in einem Abgastrakt einer Fertigungsanlage verwendet werden kann. So haben einfache Sonden zur Bestimmung spezifischer Gasanteile nicht die entsprechende Güte hinsichtlich einer Erfassungsgenauigkeit oder Erfassungsgeschwindigkeit oder sie sind allgemein zu störanfällig, um eine ausreichende Inertisierung mit ausreichender Sicherheit bestimmen zu können. Spezielle Sonden wiederum, die eine höhere Güte haben oder anderweitig an die speziellen Bedingungen in einem Abgastrakt angepasst sind, sind für eine wirtschaftliche Lösung hingegen regelmäßig zu teuer.

US 2018/239374 A1 beschreibt verschiedene Methoden zur präzisen Mischung von Gasen, bei denen die Zusammensetzung und der Druck der Gasströme kontrolliert werden. Die Verfahren beinhalten den Einsatz von Durchflussmessern, die die Strömungsraten mehrerer Gase überwachen und an ein Steuerungssystem übermitteln, das die Gase so reguliert, dass eine vorgegebene Zusammensetzung erreicht und konstant gehalten wird. Dies gilt für Anwendungen mit zwei oder mehreren Gasen, wobei das System sicherstellt, dass der Leitungsdruck auf einem vorgegebenen Niveau bleibt. Zusätzlich kann durch einen Kompositionstrim das Gasgemisch feinjustiert werden, ohne die ursprüngliche Zusammensetzung zu verändern. Die Steuerung der Gaszusammensetzung erfolgt durch eine Mischungskompositionssteuerung, die auf Basis von Echtzeitdaten kontinuierlich die Strömungsraten der einzelnen Gase anpasst. Das Konzept beschreibt sowohl "instantane" als auch "kumulative" Berechnungen der theoretischen Zusammensetzung der Gasströme und ermöglicht Alarmfunktionen, falls die gemessenen Werte außerhalb des vorgegebenen Bereich liegen. Die Systeme finden Anwendung in der Lebensmittelverarbeitung, Schweißprozessen, der Produktion von Schutzgasen und anderen industriellen Prozessen, die eine exakte Gaszusammensetzung erfordern. Die Überwachung einer Inertisierung wird jedoch nicht offenbart.

Es ist daher eine Aufgabe der vorliegenden Erfindung eine Vorrichtung und ein Verfahren anzugeben, welche(s) eine bessere Überwachung einer Inertisierung in sicherer, flexiblerer und kostengünstigerer Weise ermöglicht.

Gemäß einem Aspekt der vorliegenden Erfindung wird diese Aufgabe gelöst durch eine Vorrichtung zur Überwachung einer Inertisierung in einer Abgasabführung einer Fertigungsanlage, gemäß Anspruch 1, aufweisend: eine erste Durchflussmesseinheit; eine zweite Durchflussmesseinheit; und eine Überwachungseinheit, wobei die erste Durchflussmesseinheit mit einem ersten Durchflussmesser verbindbar ist, der in einer ersten Zuleitung eines ersten Gases zu der Fertigungsanlage angeordnet ist, wobei die zweite Durchflussmesseinheit mit einem zweiten Durchflussmesser verbindbar ist, der in einer zweiten Zuleitung eines zweiten Gases zu der Abgasabführung angeordnet ist, wobei die erste Durchflussmesseinheit dazu eingerichtet ist, anhand eines von dem ersten Durchflussmesser bereitgestellten Messwerts eine erste Gasmenge zu bestimmen, die der Fertigungsanlage zugeführt wird, wobei die zweite Durchflussmesseinheit dazu eingerichtet ist, anhand eines von dem zweiten Durchflussmesser bereitgestellten Messwerts eine zweite Gasmenge des zweiten Gases zu bestimmen, die der Abgasabführung zugeführt wird, wobei die Überwachungseinheit eingerichtet ist, eine sicherheitsgerichtete Steuerfunktion auszulösen, wenn in Abhängigkeit der bestimmten ersten Gasmenge und der bestimmten zweiten Gasmenge von einer unzureichenden Inertisierung auszugehen ist.

Gemäß einem weiteren Aspekt der vorliegenden Erfindung wird diese Aufgabe ferner gelöst durch ein Verfahren zur Überwachung einer Inertisierung in einer Abgasabführung einer Fertigungsanlage, gemäß Anspruch 12, welches folgende Schritte aufweist:
- Bereitstellen einer ersten Durchflussmesseinheit, einer zweiten Durchflussmesseinheit, sowie einer Überwachungseinheit;
- Verbinden der ersten Durchflussmesseinheit mit einem ersten Durchflussmesser, der in einer ersten Zuleitung eines ersten Gases zu der Fertigungsanlage angeordnet ist;
- Verbinden der zweiten Durchflussmesseinheit mit einem zweiten Durchflussmesser, der in einer zweiten Zuleitung eines zweiten Gases zu der Abgasabführung angeordnet ist;
- Bestimmen einer ersten Gasmenge des ersten Gases, die der Fertigungsanlage zugeführt wird, anhand eines von dem ersten Durchflussmesser bereitgestellten Messwerts;
- Bestimmen einer zweiten Gasmenge des zweiten Gases, die der Abgasabführung zugeführt wird, anhand eines von dem zweiten Durchflussmesser bereitgestellten Messwerts;
- Auslösen einer sicherheitsgerichteten Steuerfunktion, durch eine Überwachungseinheit, wenn in Abhängigkeit der bestimmten ersten Gasmenge und der bestimmten zweiten Gasmenge, von einer unzureichenden Inertisierung auszugehen ist.

Es ist somit eine Idee eine Überwachungsvorrichtung bereitzustellen, die zwei Durchflussmesseinheiten aufweist. Eine erste Durchflussmesseinheit ist mit einem Durchflussmesser innerhalb einer Zuleitung verbunden, welche ein erstes Gas für den Prozess (Prozessgas) innerhalb der Fertigungsanlage bereitstellt. Die erste Durchflussmesseinheit kann kontinuierlich eine Gasmenge erfassen, die der Fertigungsanlage zugeführt wird. Mit anderen Worten bestimmt die Durchflussmesseinheit die eingangsseitig eingeleitete Gasmenge anhand einer Durchflussmessung in einer Zuleitung des Gases zu der Fertigungsanlage. Eine zweite Durchflussmesseinheit ist mit einem Durchflussmesser verbunden, der innerhalb einer Zuleitung zu einem Abgastrakt der Fertigungsanlage angeordnet ist. Die zweite Durchflussmesseinheit kann kontinuierlich eine Inertgasmenge erfassen, die dem Abgastrakt zugeführt wird. Auch die zweite Durchflussmesseinheit bestimmt die Gasmenge anhand einer Durchflussmessung in einer Zuleitung, jedoch hier in einer Zuleitung des Inertgases.

Die beiden erfassten Gasmengen können zueinander in ein Verhältnis gesetzt werden, um hieraus Rückschlüsse auf die Inertisierung im Abgastrakt treffen zu können. Indem ein Wert für ein bestimmtes Verhältnis als Schwellwert festgelegt wird, kann eine Reaktion ausgelöst werden, falls das bestimmte Verhältnis diesen Schwellwert erreicht, insbesondere unterschreitet, und von einer unzureichenden Inertisierung auszugehen ist. Bspw. können die erfassten Gasmengen mit einer bestimmten Wasserstoff-Sauerstoff-Konzentration korrelieren und der definierte Schwellwert ein Grenzwert für eine kritische Wasserstoff-Sauerstoff-Konzentration sein, ab dem ein explosives Knallgas vorliegt.

Die Überwachung der Inertisierung in dem Abgastrakt erfolgt somit indirekt über die Bestimmung der Menge an zugeführten Gasen sowohl zur Fertigungsanlage als auch zum Abgastrakt. Dies hat den Vorteil, dass die Überwachungsvorrichtung auf einfache Durchflussmesser zurückgreifen kann. Eine tatsächliche Konzentration der verschiedenen Gase im Abgastrakt muss nicht erfasst werden. Die Durchflussmesser können einfache und bekannte Sensoren verwenden, da sie insbesondere in Zuleitungen angeordnet sein können, die ausschließlich die zu messenden Gase führen. Folglich muss nur ein Volumenstrom gemessen werden, ohne zusätzlich Art und Konzentration des Gases bestimmen zu müssen. Entsprechende Durchflussmesser unterschiedlichster Ausgestaltung sind aus dem Bereich der Prozesstechnik grundsätzlich bekannt und kostengünstig verfügbar. Die indirekte Bestimmung der Inertisierung ermöglicht somit eine kostengünstige und sichere Bestimmung einer Inertisierung auch bei intelligenten Systemen, die eine Inertisierung dynamisch einstellen. Die eingangsgenannte Aufgabe ist damit vollständig gelöst.

In einer weiteren Ausgestaltung kann die Vorrichtung ferner eine Verarbeitungseinheit aufweisen, die eingerichtet ist, aus der von der ersten Durchflussmesseinheit bestimmten ersten Gasmenge einen Sollwert für die zweite Gasmenge zu berechnen. Auf diese Weise kann unmittelbar eine Regelgröße für die Inertisierung bestimmt werden. Insbesondere kann die Überwachungseinheit eingerichtet sein, die sicherheitsgerichtete Steuerfunktion auszuführen, wenn die von der zweiten Durchflussmesseinheit bestimmte zweite Gasmenge den Sollwert unterschreitet.

Bevorzugt kann die Vorrichtung auch eine Volumenstromregeleinheit aufweisen, die mit einem Volumenstromregler verbindbar ist, der in der zweiten Zuleitung zu der Abgasabführung angeordnet ist, wobei die Volumenstromregeleinheit dazu eingerichtet ist, in Abhängigkeit der von der ersten Durchflussmesseinheit bestimmten ersten Gasmenge, d.h. anhand eines bestimmen Sollwerts für die zweite Gasmenge, eine Gaszufuhr des zweiten Gases durch die zweite Zuleitung zu regeln. Die Vorrichtung kann somit nicht nur zur Überwachung, sondern auch unmittelbar zur Regelung der Inertisierung verwendet werden. So lassen sich auch bestehende, nicht-intelligente Systeme auf einfache Weise durch die vorgeschlagene Überwachungsvorrichtung in intelligente Systeme mit dynamischer Inertisierung umwandeln. Die Überwachungsvorrichtung lässt sich auf diese Weise besonders effektiv einsetzen.

Der Volumenstromregler kann eine maximale Durchflussleistung aufweisen und ein Wert für eine maximal mögliche Fördermenge des Volumenstromreglers kann in der Volumenstromregeleinheit hinterlegbar sein. Die Verarbeitungseinheit kann eingerichtet sein, aus der von der ersten Durchflussmesseinheit bestimmten ersten Gasmenge einen Sollwert für die zweite Gasmenge zu berechnen. Ferner kann die Überwachungseinheit eingerichtet sein, die sicherheitsgerichtete Steuerfunktion auszulösen, wenn der berechnete Sollwert die maximale Durchflussleistung, d.h. eine maximal mögliche Fördermenge, übersteigt. Bei einer geregelten Inertisierung (dynamische Inertisierung) kann die Überwachung folglich an das verwendete Regelungselement ausgerichtet werden, sodass die sicherheitsgerichtete Steuerfunktion (Sicherheitsfunktion) auch dann ausgelöst wird, sollte das Regelungselement an seine Grenzen stoßen. Sollte bspw. die benötigte Menge an Inertgas eine mögliche Fördermenge, die der Volumenstromregler allein bzw. die das System einschließlich des Volumenstromreglers bereitstellen kann, überschreiten, führt dies ebenfalls zum Auslösen der Sicherheitsfunktion. Die Sicherheit des gesamten Systems lässt sich somit weiter erhöhen.

Die sicherheitsgerichtete Steuerfunktion kann eine Abschaltung einer Zufuhr des ersten Gases und/oder eine Abschaltung der Fertigungsanlage insgesamt beinhalten. Beide Maßnahmen tragen dazu bei, die Fertigungsanlage bei einer fehlerhaften oder unzureichenden Inertisierung schnell und zuverlässig in einen sicheren Zustand zu überführen. Vorteilhaft kann sich die Maßnahme auf eine einzelne Fertigungsanlage beschränken, ohne weitere Anlagen stillsetzen zu müssen, die ggf. an den gleichen Abgastrakt angeschlossen sind. Diese Ausgestaltung kann somit zu einer höheren Verfügbarkeit des Gesamtsystems vorteilhaft beitragen.

Die sicherheitsgerichtete Steuerfunktion kann auch das Einleiten einer Notspülung, insbesondere durch Öffnen eines Bypass-Ventils in der zweiten Zuleitung, beinhalten. Alternativ oder ergänzend kann die Steuerfunktion somit auch eine Notspülung ausführen und hierfür auf ein Bypass-Ventil zurückgreifen, die regelmäßig bei entsprechend Anlagen, bspw. für ein manuelle Spülung, vorhanden sind. Das Bypass-Ventil kann eine Einrichtung sein, die unabhängig von der tatsächlichen Regelung ist, um die Zufuhr auf ein Maximum der möglichen Zuführung an Inertgas einzustellen. Über diese Maßnahme lässt sich die Sicherheit weiter erhöhen.

In einer Ausgestaltung kann das erste Gas, d.h. das Prozessgas der Fertigungsanlage, Wasserstoff aufweisen und das Inertgas kann Stickstoff sein.

Ferner kann die Verarbeitungseinheit und/oder die Überwachungseinheit mehrkanalig, redundant ausgebildet sein. Auf diese Weise kann eine fehlersichere Ausführung der Überwachung und/oder der Ausführung des sicherheitsgerichteten Steuerfunktion gewährleistet werden, um normative Vorgaben zu erfüllen. Ergänzend können die Verarbeitungseinheit und die Überwachungseinheit auch einen diversitären Aufbau mit unterschiedlicher Hardware aufweisen. Die redundante Auslegung ermöglicht es, ständige Tests der Ein- und Ausgänge sowie einen ständigen Vergleich der Anwenderdaten vorzunehmen, um die eigene Fehlersicherheit zu gewährleisten.

Es versteht sich, dass die voranstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert.
Fig. 1 zeigt in einer schematischen Darstellung eine Fertigungsanlage mit einem Bespiel einer Überwachungsvorrichtung zur Überwachung einer Inertisierung.
Fig. 2 zeigt verschiedene Weiterbildungen der Überwachungsvorrichtung gemäß Fig. 1.
Fig. 3 zeigt in einer schematischen Darstellung ein Beispiel eines Verfahrens zur Überwachung einer Inertisierung.

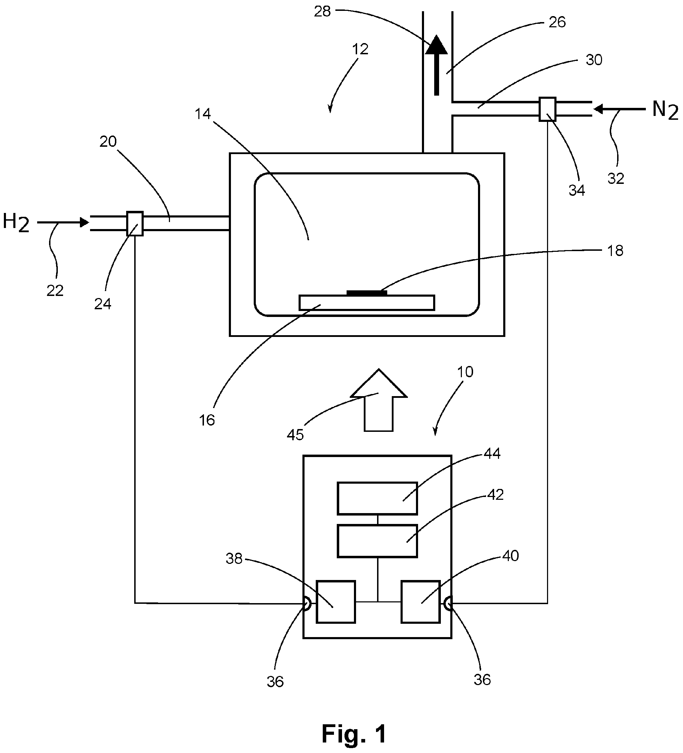

Fig. 1 zeigt eine Fertigungsanlage mit einem Bespiel einer Überwachungsvorrichtung. Die Überwachungsvorrichtung ist hier in ihrer Gesamtheit mit der Bezugsziffer 10 bezeichnet.

Die Fertigungsanlage 12 ist exemplarisch eine Fertigungsanlage aus dem Bereich der Halbleiterfertigung und hier stark vereinfacht dargestellt. Die Fertigungsanlage 12 verfügt mindestens über eine Kammer 14, innerhalb derer der Fertigungsprozess abläuft. Für die Fertigung kann bspw. auf einen Objektträger 16 in der Kammer 14 ein Halbleitersubstrat 18 gelegt sein, an dem verschiedene mechanische und chemische Fertigungsschritte durchführbar sind, um eine bestimmtes Halbleiterelement zu fertigen.

Die Fertigungsanlage 12 verfügt mindestens über eine Zuleitung 20, über die ein Prozessgas 22 für die Fertigung in der Kammer 14 hinzugefügt werden kann. Das Prozessgas 22 kann unmittelbar für die chemische Bearbeitung des Halbleiterbauelements verwendet werden oder aber ein Prozessgas sein, dass dazu beiträgt, innerhalb der Kammer 14 eine für die Fertigung notwendige Arbeitsumgebung zu erzeugen. Wie hier angedeutet, kann das Prozessgas 22 (erstes Gas) beispielsweise Wasserstoff H₂ sein.

In oder an der Zuleitung 20 des Prozessgases 22 ist zudem ein Durchflussmesser 24 angeordnet. Der Durchflussmesser 24 kann verschiedenartig ausgebildet sein und einen Wert, der eine Gaszufuhr des Prozessgases repräsentiert, bereitstellen. Der Durchflussmesser 24 kann insbesondere ein einfacher Volumenstrommesser sein, der eine Gaszufuhr anhand eines Volumenstroms bestimmt, vorzugsweise ohne eine Konzentration des entsprechenden Gases selbst bestimmen zu müssen.

Ferner verfügt die Fertigungsanlage 12 über einen Abgastrakt 26 (Abgasabführung), über den die in die Kammer 14 eingeführten Prozessgase bzw. etwaige Reaktionsprodukte aus der Kammer 14 abgeführt werden können. Die abgeführten Prozessgase bzw. Reaktionsprodukte werden im Folgenden kurz als Abgase 28 bezeichnet. Die Abgase 28 können aktiv oder passiv aus der Kammer 14 in den Abgastrakt 26 herausgeführt werden.

An den Abgastrakt 26 ist eine weitere Zuleitung 30 angeordnet, vorzugsweise unmittelbar nach der Herausführung des Abgastrakts 26 aus der Fertigungsanlage 12. Über die weitere Zuleitung 30 kann ein Inertgas 32 dem Abgastrakt 26 zugeführt werden, um eine Inertisierung im Abgastrakt 26 durchzuführen. Mit anderen Worten, in den Abgastrakt 26 wird ein Inertgas eingeführt, um die Abgase 28 zu verdünnen bzw. um bestimmte Gase, die im Abgastrakt vorhanden sein können, zu verdrängen. Das Inertgas 32 kann, wie hier angedeutet, beispielsweise Stickstoff N₂ sein.

Wie in der Zuleitung 20 für das Prozessgas 22, ist auch in oder an der weiteren Zuleitung 30 ein Durchflussmesser 34 angeordnet. Der weitere Durchflussmesser 34 kann einen Wert bereitstellen, der einer Gaszufuhr an Inertgas entspricht. Wie bei dem Durchflussmesser 24 in der ersten Zuleitung 20 kann auch der weitere Durchflussmesser 34 ein einfacher Volumenstrommesser sein.

Die Werte des Durchflussmessers 24 und des weiteren Durchflussmessers 34 werden der Überwachungsvorrichtung 10 zugeführt, die hierfür entsprechende Eingangsanschlüsse 36 aufweist. Eine erste Durchflussmesseinheit 38 innerhalb der Überwachungsvorrichtung 10 kann anhand des von dem ersten Durchflussmesser 24 bereitgestellten Werts kontinuierlich eine Prozessgasmenge (erste Gasmenge) bestimmen, die der Kammer 14 zugeführt worden ist. Gleichzeitig kann eine zweite Durchflussmesseinheit 40 innerhalb der Überwachungsvorrichtung 10 eine Inertgasmenge (zweite Gasmenge) bestimmen, die dem Abgastrakt 26 zugeführt worden ist. Eine Verarbeitungseinheit 42 kann die von den Durchflussmesseinheiten 38, 40 bestimmten Gasmengen in ein definiertes Verhältnis zueinander setzen. Das definierte Verhältnis kann einen Grad der Inertisierung innerhalb des Abgastrakts 26, der sich aus den bereitgestellten Gasmengen ergibt, repräsentieren. Denkbar ist beispielsweise, dass die bereitgestellten Gasmengen unmittelbar mit einer Wasserstoff-Sauerstoff-Konzentration in dem Abgastrakt 26 korreliert. Es versteht sich, dass für die Bestimmung des definierten Verhältnisses die Verarbeitungseinheit 42 mindestens einen weiteren Parameter berücksichtigen kann bzw. Korrekturfaktoren in die Bestimmung mit einfließen lässt, die für die Fertigungsanlage 12, den Abgastrakt 26 oder den Fertigungsprozess spezifisch sind. Auf diese Weise lässt sich die Überwachungsvorrichtung flexibel an verschiedene Anlagen anpassen.

Eine Überwachungseinheit 44 der Überwachungsvorrichtung 10 kann kontinuierlich das von der Verarbeitungseinheit 42 bestimmte Verhältnis mit einem definierten Schwellwert vergleichen. Die Überwachungseinheit 44 löst eine sicherheitsgerichtete Steuerfunktion aus, wenn das bestimmte Verhältnis den Schwellwert erreicht. Bspw. löst die Überwachungseinheit 44 die sicherheitsgerichtete Steuerfunktion aus, wenn das bestimmte Verhältnis den Schwellwert unterschreitet und infolgedessen von einer unzureichenden in Inertisierung im Abgastrakt 26 auszugehen ist. Die Überwachungseinheit 44 löst somit die sicherheitsgerichtete Steuerfunktion in Abhängigkeit der bestimmten Gasmengen aus.

Die sicherheitsgerichtete Steuerfunktion 45 dient dazu, eine von der unzureichenden Inertisierung ausgehende Gefahr zu reduzieren. Die sicherheitsgerichtete Steuerfunktion 45 kann auf unterschiedliche Weise, je nach Art der Fertigungsanlage 12 und des von der Fertigungsanlage 12 ausgeführten Prozesses, ausgebildet sein. In einer Ausführungsform kann die sicherheitsgerichtete Funktion 45 bspw. die Abschaltung einer Gaszufuhr des Prozessgases in der Zuleitung 20 beinhalten. Alternativ oder ergänzend kann die sicherheitsgerichtete Funktion 45 auch ein Abschalten der Fertigungsanlage 12 an sich zur Folge haben. Wiederum in einer anderen Ausführungsform kann die sicherheitsgerichtete Steuerfunktion 45 auch eine aktive Maßnahme beinhalten, wie beispielsweise das Auslösen einer separaten Spülung des Abgastrakts 26. Für eine solche Spülung kann ein Bypass-Ventil (hier nicht dargestellt) vorgesehen sein, das unabhängig von einer Regelung der Inertgasmenge, einen maximalen Zufluss an Inertgas in den Abgastrakt 26 ermöglicht.

Es versteht sich, dass die vorstehend beschriebenen Einheiten, insbesondere die Aufteilung in die verschiedenen Einheiten, rein funktional zu verstehen sind, und die individuellen Einheiten auch in eine oder mehrere Komponenten integriert sein können. In einer Ausführungsform können beispielsweise die Durchflussmesseinheiten 38, 40 die Verarbeitungseinheit 42, und die Überwachungseinheit 44 durch eine zentrale Verarbeitungseinheit, wie bspw. einen zentralen Prozessor (CPU), einen ASIC oder einen Mikrocontroller, realisiert sein.

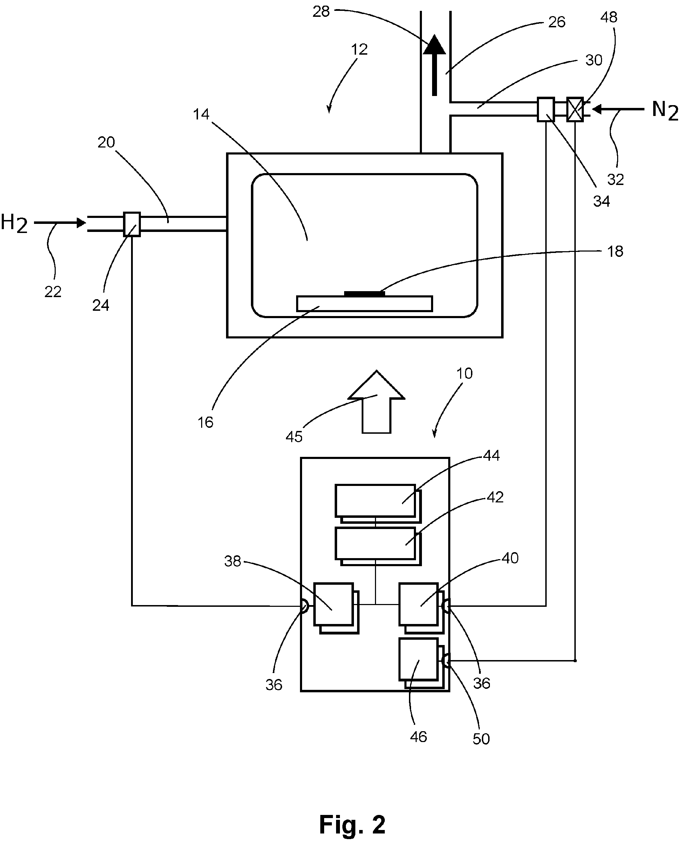

Vorzugsweise sind die einzelnen Einheiten innerhalb eines gemeinsamen Gehäuses zu einer einzelnen funktionalen Steuervorrichtung integriert, die in einem Schaltschrank der Fertigungsanlage 12 anordenbar ist. Die Steuervorrichtung kann eine modulare Steuervorrichtung sein, die sich aus einzelnen Hardware- und Softwaremodulen, die die verschiedenen Aufgaben der oben beschriebenen Einheiten implementieren, zusammensetzt. Die modulare Steuervorrichtung kann eine Kommunikationseinrichtung, bspw. einen die einzelnen Module verbindenden Modulbus, aufweisen, über den die Einheiten kommunikativ miteinander verbunden sind. Die Steuervorrichtung kann ferner erweiterbar sein und weitere Steuer- und Regelungsaufgaben wahrnehmen. Beispiele für Weiterbildungen der in Fig. 1 gezeigten Überwachungsvorrichtung werden mit Bezug auf die Fig. 2 im Folgenden näher erläutert.

Fig. 2 zeigt beispielhaft verschiedene Weiterbildungen der zuvor beschriebenen Überwachungsvorrichtung 10. Gleiche Bezugszeichen bezeichnen hierbei gleiche Teile wie zuvor.

Die Überwachungsvorrichtung 10 gemäß Fig. 2 unterscheidet sich insbesondere durch eine zusätzliche Volumenstromregeleinheit 46 von der Überwachungsvorrichtung 10 gemäß Fig. 1. Die Volumenstromregeleinheit 46 ist mit einem Volumenstromregler 48 verbindbar, der in der zweiten Zuleitung 30 stromaufwärts, d. h. in Flussrichtung vor dem zweiten Volumenstrommesser 34, angeordnet ist. Der Volumenstromregler 48 kann ein regelbares Ventil (engl. Mass Flow Controller (MFC) sein. In einer Ausführungsform kann der Volumenstromregler 48 beispielsweise ein Regelventil mit einem Medium-getrennten Sensor und einem integrierten Proportional-Integral-(PI)-Regler sein. Die Erfindung ist jedoch nicht auf eine bestimmte Art von Regler beschränkt. Der Volumenstromregler 48 muss lediglich dazu geeignet sein, einen Volumenstrom innerhalb der zweiten Zuleitung regulieren zu können. Der Volumenstromregler 48 ist daher auch nicht auf eine einzelne Einrichtung, wie in Fig. 2 dargestellt, beschränkt, sondern kann eine Kombination einer Vielzahl verschiedener Komponenten beinhalten, die zusammen den Volumenstrom regulieren.

Die Ansteuerung für die Regelung erfolgt durch die Volumenstromregeleinheit 46 der Überwachungsvorrichtung 10. Die Volumenstromregeleinheit 46 kann in gleicher Weise in die Überwachungsvorrichtung 10 integriert sein, wie die zuvor beschriebenen Volumenstrommesseinheiten 38, 40 mit dem Unterschied, dass die Volumenstromregeleinheit 46 mit mindestens einem Ausgang 50 der Überwachungsvorrichtung 10 gekoppelt ist, über den die Volumenstromregeleinheit 46 ein Steuersignal an den Volumenstromregler 48 übertragen kann.

Die Volumenstromregeleinheit 46 kann den Volumenstromregler 48 gemäß einer Vorgabe der Verarbeitungseinheit 42 ansteuern. Die Vorgabe wiederum basiert auf einem Wert für die Gasmenge, die der Fertigungsanlage 12 über die erste Zuleitung 20 zugeführt wird, und basiert somit auf dem durch die erste Volumenstrommesseinheiten 38 ermittelten Wert für die Menge an zugeführten Prozessgas 22. Die Vorgabe muss nicht direkt dem Messwert entsprechen, sondern kann bspw. zusätzliche Korrekturfaktoren, Toleranzen oder anderweitig Umformung berücksichtigen. Die Vorgabe regelt die Zuführung von Inertgas 32 durch die zweite Zuleitung 30 so, dass eine ausreichende Inertisierung im Abgastrakt 26 gewährleistet wird. Mit anderen Worten, die Überwachungsvorrichtung 10 stellt die Menge an Inertgas dynamisch auf eine benötigte Menge ein. Hierfür kann die Verarbeitungseinheit 42 anhand der eingeleiteten Menge an Prozessgas 22, einen Sollwert für die Menge an Inertgas 32 bestimmen, die für eine ausreichende Verdünnung bzw. Verdrängung im Abgastrakt 26 benötigt wird. Anhand des Sollwerts wird über die Volumenstromregeleinheit 46 der Volumenstromregler 48 geregelt und der Zufluss an Inertgas reguliert.

In einem konkreten Beispiel darf bei der Verwendung von Wasserstoff als Prozessgas 22 bspw. ein Volumenanteil von Wasserstoff im Abgastrakt nicht größer als 3vol% sein. Aus dem Wissen über die eingeleitete Menge an Wasserstoff und unter der Annahme, dass hier von bis zu 100% in den Abgastrakt abgeführt werden können, kann die Verarbeitungseinheit 42, gegebenenfalls unter Berücksichtigung weiterer Parameter, eine Menge an Inertgas 32 bestimmen, die notwendig ist, um den abgeleiteten Wasserstoff im Abgastrakt 26 ausreichend zu verdünnen, sodass der kritische Volumenanteil nicht überschritten wird. Über die Volumenstromregeleinheit 46 kann anschließend die Zuführung des Inertgases gesteuert werden. Neben dieser Regelung kann die Überwachungsvorrichtung 10 die zuvor in Bezug auf Fig. 1 beschriebene Überwachung der Inertisierung durchführen und eine sicherheitsgerichtete Reaktion auslösen, wenn von einer unzureichenden Inertisierung auszugehen ist.

Bei der Überwachungsvorrichtung 10 gemäß Figur 2 kann zusätzlich zu der zuvor beschriebenen Überwachung der Inertisierung ergänzend auch die Regelung selbst überwacht werden. Beispielsweise kann die Überwachungseinheit 44 die sicherheitsgerichtete Reaktion auch dann auslösen, wenn ein von der Verarbeitungseinheit 42 bestimmter Sollwert einen maximal möglichen Regelzustand überschreitet. Der maximal mögliche Regelzustand kann beispielsweise durch eine maximale Durchflussmenge des Volumenstromreglers 48, den maximal möglichen Einspeisedruck/-durchmesser etc. begrenzt sein und sich auf die Menge an Inertgas beziehen, die mittels des Volumenstromregler 48 maximal durch die Zuleitung 30 förderbar ist. In diesem Fall kann die sicherheitsgerichtete Reaktion auch dann ausgelöst werden, wenn der notwendige Sollwert nicht erreicht werden kann. Darüber hinaus können auch weitere Faktoren, die die Inertisierungsmöglichkeiten beschränken, durch zusätzliche Schwellwerte berücksichtigt werden.

Die Überwachungsvorrichtung 10 kann insbesondere eine Sicherheitssteuerung sein. Eine Sicherheitssteuerung kann Regelaufgaben in gleicher Weise ausführen, wie eine normale Steuerung, beispielsweise eine programmierbare Steuerung (SPS), der Automatisierungstechnik. Im Gegensatz dazu verfügt eine Sicherheitssteuerung jedoch über zusätzliche Soft- und Hardwareeinrichtungen, die eine fehlersichere Ausführung bestimmter Steuer- und Regelfunktionen gewährleisten können. Zu den zusätzlichen Einrichtungen zählen bspw. eine mehrkanalige, redundante Auslegung der wesentlichen Verarbeitungseinheiten und Schnittstellen der Überwachungsvorrichtung 10. Dies ist in der Fig. 2 durch die jeweils doppelte Darstellung der einzelnen Einheiten angedeutet.

Neben einer redundanten Auslegung der einzelnen funktionalen Komponenten der Sicherheitssteuerung können die Komponenten auch diversitär ausgestaltet sein, bspw. in dem die redundanten Komponenten von unterschiedlichen Herstellern bezogen werden. Auf diese Weise können auch Fehler mit gemeinsamer Ursache effektiv ausgeschlossen werden, wodurch die Eigenfehlersicherheit weiter erhöht wird.

Neben der vorstehend beschriebenen Überwachung der Inertisierung und deren Regelung können durch die Verwendung einer Sicherheitssteuerung vorteilhaft weitere Aspekte der Reglung wie bspw. Toleranzfenster, Toleranzzeiten oder Totzeiten fehlersicher überwacht werden und damit zu einem Teil des Sicherheitskreises werden. Gleiches gilt für Korrekturen von Unsicherheiten durch Abweichungen in der Hardwareausführung der Messwerterfassung, die mittels einer Sicherheitssteuerung ebenfalls sicherheitsgerichtet ausgeführt werden können. Die Sicherheit kann auf diese Weise weiter erhöht werden.

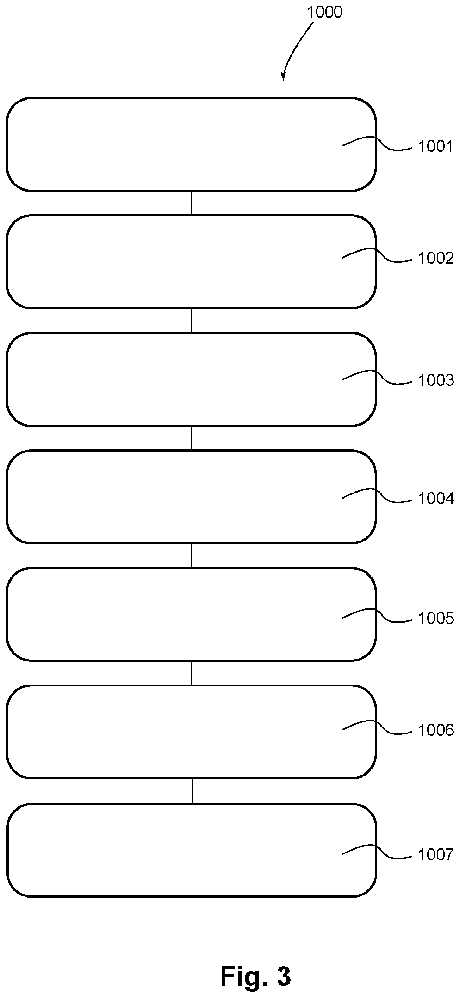

Mit Bezug auf Fig. 3 wird im Folgenden ein Beispiel für ein Verfahren zur Überwachung einer Inertisierung beschrieben. Das Verfahren lässt sich zusammenfassen als ein Verfahren, bei dem zur Überwachung einer Inertisierung in einem Abgastrakt, eine Menge an einer Fertigungsanlage zugeführten Prozessgas und eine Menge an dem Abgastrakt zugeführten Inertgas kontinuierlich bestimmt wird, um ein Mengenverhältnis dieser beiden Gase im Abgastrakt rechnerisch zu bestimmen sowie in Abhängigkeit davon eine sicherheitsgerichtete Steuerfunktion auszulösen, wenn das Mengenverhältnis einen kritischen Wert (Schwellwert) annimmt.

Im Einzelnen kann das Verfahren die in Fig. 3 angedeuteten Schritte aufweisen. Das Verfahren ist in seiner Gesamtheit mit der Bezugsziffer 1000 bezeichnet.

In einem ersten Schritt 1001 wird eine Überwachungsvorrichtung bereitgestellt. Die Überwachungsvorrichtung weist eine Verarbeitungseinheit, eine erste Durchflussmesseinheit, eine zweite Durchflussmesseinheit, sowie eine Überwachungseinheit auf.

Anschließend (Schritt 1002) wird die erste Durchflussmesseinheit mit einem ersten Durchflussmesser verbunden, der in einer ersten Zuleitung eines ersten Gases zu der Fertigungsanlage angeordnet ist.

Ferner wird die zweite Durchflussmesseinheit mit einem zweiten Durchflussmesser verbunden, der in einer zweiten Zuleitung eines zweiten Gases zu der Abgasabführung angeordnet ist (Schritt 1003).

Danach erfolgt das Bestimmen einer ersten Gasmenge des ersten Gases, die der Fertigungsanlage beim Betrieb der Anlage zugeführt wird, anhand eines von dem ersten Durchflussmesser bereitgestellten Messwerts (Schritt 1004) sowie das Bestimmen einer zweiten Gasmenge des zweiten Gases, die der Abgasabführung zugeführt wird, anhand eines von dem zweiten Durchflussmesser bereitgestellten Messwerts (Schritt 1005). Die Bestimmung wird vorzugweise kontinuierlich ab dem Start der Fertigungsanlage durchgeführt.

Schließlich wird anhand der ermittelten Gasmengen bestimmt, in welchem Verhältnis diese zueinander im Abgastrakt vorliegen können (Schritt 1006), ohne die tatsächliche Konzentration der jeweiligen Gase in der Abgasabführung explizit zu messen. Ferner wird im Schritt 1007 eine sicherheitsgerichtete Steuerfunktion in Abhängigkeit der ermittelten Gasmengen ausgelöst, d.h. die Überwachungseinheit löst eine sicherheitsgerichtete Steuerfunktion aus, falls das Verhältnis einen kritischen Schwellwert erreicht. Der Schwellwert kann hierbei vorab rechnerisch oder empirisch ermittelt worden sein, oder aber dynamisch eingeregelt werden.

Es versteht sich, dass das Verfahren nicht auf die hier gezeigte Ausführung beschränkt ist, sondern noch weitere Schritte beinhalten kann, die vor oder nach den einzelnen Schritten eingefügt sind. Ebenso können einzelne Schritte wiederholt, insbesondere kontinuierlich ausgeführt werden, ohne dass dies hier explizit angedeutet ist.

Grundsätzlich ist die vorliegende Erfindung nicht durch die hier aufgeführten Ausführungsbeispiele beschränkt, sondern allein durch die nachfolgenden Ansprüche definiert.

## Patentansprüche

1. Vorrichtung (10) zur Überwachung einer Inertisierung in einer Abgasabführung (26) einer Fertigungsanlage (12), aufweisend:
eine erste Durchflussmesseinheit (38);
eine zweite Durchflussmesseinheit (40); und
eine Überwachungseinheit (44),
wobei die erste Durchflussmesseinheit (38) mit einem ersten Durchflussmesser (24) verbindbar ist, der in einer ersten Zuleitung (20) eines ersten Gases zu der Fertigungsanlage (12) angeordnet ist,
wobei die zweite Durchflussmesseinheit (40) mit einem zweiten Durchflussmesser (34) verbindbar ist, der in einer zweiten Zuleitung (30) eines zweiten Gases zu der Abgasabführung (26) angeordnet ist,
wobei die erste Durchflussmesseinheit (38) dazu eingerichtet ist, anhand eines von dem ersten Durchflussmesser (24) bereitgestellten Messwerts eine erste Gasmenge des ersten Gases zu bestimmen, die der Fertigungsanlage (12) zugeführt wird,
wobei die zweite Durchflussmesseinheit (40) dazu eingerichtet ist, anhand eines von dem zweiten Durchflussmesser (34) bereitgestellten Messwerts eine zweite Gasmenge des zweiten Gases zu bestimmen, die der Abgasabführung (26) für die Inertisierung zugeführt wird, und
wobei die Überwachungseinheit (44) eingerichtet ist, eine sicherheitsgerichtete Steuerfunktion (45) auszulösen, wenn in Abhängigkeit der bestimmten ersten Gasmenge und der bestimmten zweiten Gasmenge von einer unzureichenden Inertisierung auszugehen ist.

2. Vorrichtung nach Anspruch 1, ferner aufweisend: eine Verarbeitungseinheit (42), die eingerichtet ist, aus der von der ersten Durchflussmesseinheit (38) bestimmten ersten Gasmenge einen Sollwert für die zweite Gasmenge zu berechnen.

3. Vorrichtung nach Anspruch 2, wobei die Überwachungseinheit (44) eingerichtet ist, die sicherheitsgerichtete Steuerfunktion (45) auszulösen, wenn die von der zweiten Durchflussmesseinheit (40) bestimmte zweite Gasmenge den von der Verarbeitungseinheit (42) bestimmten Sollwert unterschreitet.

4. Vorrichtung nach einem der Ansprüche 1 bis 3, ferner aufweisend: eine Volumenstromregeleinheit (46), die mit einem Volumenstromregler (48) verbindbar ist, der in der zweiten Zuleitung (30) zu der Abgasabführung (26) angeordnet ist, wobei die Volumenstromregeleinheit (46) dazu eingerichtet ist, in Abhängigkeit der von der ersten Durchflussmesseinheit (38) bestimmten ersten Gasmenge eine Gaszufuhr des zweiten Gases durch die zweite Zuleitung (30) zu regeln.

5. Vorrichtung nach Anspruch 4, wobei der Volumenstromregler (48) auf eine definierte maximale Fördermenge einregelbar ist, deren Wert in der Volumenstromregeleinheit (46) hinterlegbar ist, und wobei die Vorrichtung eine Verarbeitungseinheit (42) aufweist, die eingerichtet ist, aus der von der ersten Durchflussmesseinheit (38) bestimmten ersten Gasmenge einen Sollwert für die zweite Gasmenge zu berechnen, und wobei die Überwachungseinheit (44) eingerichtet ist, die sicherheitsgerichtete Steuerfunktion auszulösen, wenn der berechnete Sollwert den Wert für die maximale Fördermenge übersteigt.

6. Vorrichtung nach einem der Ansprüche 1 bis 5, wobei die sicherheitsgerichtete Steuerfunktion (45), eine Abschaltung einer Zufuhr des ersten Gases und/oder eine Abschaltung der Fertigungsanlage (12) beinhaltet.

7. Vorrichtung nach einem der Ansprüche 1 bis 6, wobei die sicherheitsgerichtete Steuerfunktion (45) das Einleiten einer Notspülung beinhaltet.

8. Vorrichtung nach Anspruch 7, wobei die Notspülung das Öffnen eines Bypass-Ventils in der zweiten Zuleitung (30) beinhaltet.

9. Vorrichtung nach einem der Ansprüche 1 bis 8, wobei das erste Gas Wasserstoff enthält.

10. Vorrichtung nach einem der Ansprüche 1 bis 8, wobei das zweite Gas ein Inertgas ist.

11. Vorrichtung nach Anspruch 10, wobei das Inertgas Stickstoff ist.

12. Verfahren (1000) zur Überwachung einer Inertisierung in einer Abgasabführung (26) einer Fertigungsanlage (12), aufweisend:
- Bereitstellen einer ersten Durchflussmesseinheit, einer zweiten Durchflussmesseinheit sowie einer Überwachungseinheit (1001);
- Verbinden der ersten Durchflussmesseinheit mit einem ersten Durchflussmesser, der in einer ersten Zuleitung eines ersten Gases zu der Fertigungsanlage angeordnet ist (1002);
- Verbinden der zweiten Durchflussmesseinheit mit einem zweiten Durchflussmesser, der in einer zweiten Zuleitung eines zweiten Gases zu der Abgasabführung angeordnet ist (1003);
- Bestimmen einer ersten Gasmenge des ersten Gases, die der Fertigungsanlage zugeführt wird, anhand eines von dem ersten Durchflussmesser bereitgestellten Messwerts (1004);
- Bestimmen einer zweiten Gasmenge des zweiten Gases, die der Abgasabführung für die Inertisierung zugeführt wird, anhand eines von dem zweiten Durchflussmesser bereitgestellten Messwerts (1005); und
- Auslösen einer sicherheitsgerichteten Steuerfunktion, durch eine Überwachungseinheit (44), wenn in Abhängigkeit der bestimmten ersten Gasmenge und der bestimmten zweiten Gasmenge (1007) von einer unzureichenden Inertisierung auszugehen ist.

## Claims

1. Apparatus (10) for monitoring an inertization in an exhaust gas discharge (26) of a production plant (12), comprising:
a first flow measurement unit (38);
a second flow measurement unit (40);
and a monitoring unit (44),
wherein the first flow measurement unit (38) is connectable to a first flow meter (24), which is arranged in a first supply line (20) of a first gas to the production plant (12),
wherein the second flow measurement unit (40) is connectable to a second flow meter (34), which is arranged in a second supply line (30) of a second gas to the exhaust gas discharge (26),
wherein the first flow measurement unit (38) is configured to determine, on the basis of a measured value provided by the first flow meter (24), a first gas quantity of the first gas which is supplied to the production plant (12),
wherein the second flow measurement unit (40) is configured to determine, on the basis of a measured value provided by the second flow meter (34), a second gas quantity of the second gas which is supplied to the exhaust gas discharge (26) for the inertization, and
wherein the monitoring unit (44) is configured to trigger a safety-related control function (45) if, depending on the determined first gas quantity and the determined second gas quantity, an insufficient inertization is to be assumed.

2. Apparatus according to claim 1, further comprising: a processing unit (42) which is configured to calculate a target value for the second gas quantity from the first gas quantity determined by the first flow measurement unit (38).

3. Apparatus according to claim 2, wherein the monitoring unit (44) is configured to trigger the safety-related control function (45) if the second gas quantity determined by the second flow measurement unit (40) falls below the target value determined by the processing unit (42).

4. Apparatus according to one of claims 1 to 3, further comprising: a volume flow control unit (46) which is connectable to a volume flow controller (48), which is arranged in the second supply line (30) to the exhaust gas discharge (26), wherein the volume flow control unit (46) is configured to control a gas supply of the second gas through the second supply line (30) depending on the first gas quantity determined by the first flow measurement unit (38).

5. Apparatus according to claim 4, wherein the volume flow controller (48) is adjustable to a defined maximum delivery quantity, the value of which can be stored in the volume flow control unit (46), and wherein the apparatus comprises a processing unit (42) which is configured to calculate a target value for the second gas quantity from the first gas quantity determined by the first flow measurement unit (38), and wherein the monitoring unit (44) is configured to trigger the safety-related control function if the calculated target value exceeds the value for the maximum delivery quantity.

6. Apparatus according to one of claims 1 to 5, wherein the safety-related control function (45) includes a shutdown of a supply of the first gas and/or a shutdown of the production plant (12).

7. Apparatus according to one of claims 1 to 6, wherein the safety-related control function (45) includes the initiation of an emergency purge.

8. Apparatus according to claim 7, wherein the emergency purge includes the opening of a bypass valve in the second supply line (30).

9. Apparatus according to one of claims 1 to 8, wherein the first gas contains hydrogen.

10. Apparatus according to one of claims 1 to 8, wherein the second gas is an inert gas.

11. Apparatus according to claim 10, wherein the inert gas is nitrogen.

12. Method (1000) for monitoring an inertization in an exhaust gas discharge (26) of a production plant (12), comprising:
- providing a first flow measurement unit, a second flow measurement unit as well as a monitoring unit (1001); - connecting the first flow measurement unit to a first flow meter, which is arranged in a first supply line of a first gas to the production plant (1002);
- connecting the second flow measurement unit to a second flow meter, which is arranged in a second supply line of a second gas to the exhaust gas discharge (1003);
- determining a first gas quantity of the first gas which is supplied to the production plant, on the basis of a measured value provided by the first flow meter (1004);
- determining a second gas quantity of the second gas which is supplied to the exhaust gas discharge for the inertization, on the basis of a measured value provided by the second flow meter (1005); and
- triggering a safety-related control function, by a monitoring unit (44), if, depending on the determined first gas quantity and the determined second gas quantity (1007), an insufficient inertization is to be assumed.

## Revendications

1. Dispositif (10) pour la surveillance d'un inertage dans une évacuation de gaz d'échappement (26) d'une installation de fabrication (12), présentant :
une première unité de mesure de débit (38) ;
une seconde unité de mesure de débit (40) ; et
une unité de surveillance (44),
dans lequel la première unité de mesure de débit (38) peut être connectée à un premier débitmètre (24) qui est disposé dans une première conduite d'alimentation (20) d'un premier gaz vers l'installation de fabrication (12),
dans lequel la seconde unité de mesure de débit (40) peut être connectée à un second débitmètre (34) qui est disposé dans une seconde conduite d'alimentation (30) d'un second gaz vers l'évacuation de gaz d'échappement (26),
dans lequel la première unité de mesure de débit (38) est configurée pour déterminer, à l'aide d'une valeur de mesure fournie par le premier débitmètre (24), une première quantité de gaz du premier gaz qui est amenée à l'installation de fabrication (12),
dans lequel la seconde unité de mesure de débit (40) est configurée pour déterminer, à l'aide d'une valeur de mesure fournie par le second débitmètre (34), une seconde quantité de gaz du second gaz qui est amenée à l'évacuation de gaz d'échappement (26) pour l'inertage, et
dans lequel l'unité de surveillance (44) est configurée pour déclencher une fonction de commande de sécurité (45) si, en fonction de la première quantité de gaz déterminée et de la seconde quantité de gaz déterminée, l'inertage est supposé insuffisant.

2. Dispositif selon la revendication 1, présentant en outre : une unité de traitement (42) qui est configurée pour calculer une valeur de consigne pour la seconde quantité de gaz à partir de la première quantité de gaz déterminée par la première unité de mesure de débit (38).

3. Dispositif selon la revendication 2, dans lequel l'unité de surveillance (44) est configurée pour déclencher la fonction de commande de sécurité (45) lorsque la seconde quantité de gaz déterminée par la seconde unité de mesure de débit (40) est inférieure à la valeur de consigne déterminée par l'unité de traitement (42).

4. Dispositif selon l'une des revendications 1 à 3, présentant en outre : une unité de régulation de débit volumique (46) qui peut être connectée à un régulateur de débit volumique (48) qui est disposé dans la seconde conduite d'alimentation (30) vers l'évacuation de gaz d'échappement (26), dans lequel l'unité de régulation de débit volumique (46) est configurée pour réguler une amenée en gaz du second gaz à travers la seconde conduite d'alimentation (30) en fonction de la première quantité de gaz déterminée par la première unité de mesure de débit (38).

5. Dispositif selon la revendication 4, dans lequel le régulateur de débit volumique (48) peut être réglé sur un débit maximal défini dont la valeur peut être mémorisée dans l'unité de régulation de débit volumique (46), et dans lequel le dispositif présente une unité de traitement (42) qui est configurée pour calculer une valeur de consigne pour la seconde quantité de gaz à partir de la première quantité de gaz déterminée par la première unité de mesure de débit (38), et dans lequel l'unité de surveillance (44) est configurée pour déclencher la fonction de commande de sécurité lorsque la valeur de consigne calculée dépasse la valeur pour le débit maximal.

6. Dispositif selon l'une des revendications 1 à 5, dans lequel la fonction de commande de sécurité (45) comprend une coupure d'une amenée du premier gaz et/ou une coupure de l'installation de fabrication (12).

7. Dispositif selon l'une des revendications 1 à 6, dans lequel la fonction de commande de sécurité (45) comprend le déclenchement d'un rinçage d'urgence.

8. Dispositif selon la revendication 7, dans lequel le rinçage d'urgence comprend l'ouverture d'une soupape de dérivation dans la seconde conduite d'alimentation (30).

9. Dispositif selon l'une des revendications 1 à 8, dans lequel le premier gaz contient de l'hydrogène.

10. Dispositif selon l'une des revendications 1 à 8, dans lequel le second gaz est un gaz inerte.

11. Dispositif selon la revendication 10, dans lequel le gaz inerte est de l'azote.

12. Procédé (1000) pour la surveillance d'un inertage dans une évacuation de gaz d'échappement (26) d'une installation de fabrication (12), présentant :
- la fourniture d'une première unité de mesure de débit, d'une seconde unité de mesure de débit ainsi que d'une unité de surveillance (1001) ;
- la connexion de la première unité de mesure de débit à un premier débitmètre qui est disposé dans une première conduite d'alimentation d'un premier gaz vers l'installation de fabrication (1002) ;
- la connexion de la seconde unité de mesure de débit à un second débitmètre qui est disposé dans une seconde conduite d'alimentation d'un second gaz vers l'évacuation de gaz d'échappement (1003) ;
- la détermination d'une première quantité de gaz du premier gaz qui est amenée à l'installation de fabrication à partir d'une valeur de mesure fournie par le premier débitmètre (1004) ;
- la détermination d'une seconde quantité de gaz du second gaz qui est amenée à l'évacuation de gaz d'échappement pour l'inertage à l'aide d'une valeur de mesure fournie par le second débitmètre (1005) ; et
- le déclenchement d'une fonction de commande de sécurité par une unité de surveillance (44) si, en fonction de la première quantité de gaz déterminée et de la seconde quantité de gaz déterminée, l'inertage est supposé insuffisant (1007).
